# EUROPEAN PATENT SPECIFICATION

(11) **EP 1 416 810 B1**
(45) Date of publication and mention of the grant of the patent: **15.04.2009**
(21) Application number: 02767339.1
(22) Date of filing: 07.08.2002
(51) Int. Cl.: A23L 1/0524, A23L 1/275

(54) **COMPOSITIONS COMPRISING SUGAR BEET PECTIN AND CAROTENOIDS**
ZUSAMMENSETZUNGEN ENTHALTEND ZUCKERRÜBEN PEKTIN UND CAROTINOIDE
COMPOSITIONS COMPRENANT DE LA PECTINE DE BETTERAVE A SUCRE ET DES CAROTENOIDES

(30) Priority: 13.08.2001 EP 01119429
(43) Date of publication of application: 12.05.2004
(73) Proprietor: DSM IP Assets B.V., 6411 TE Heerlen (NL)
(72) Inventor: BECK, Markus, Ivo, 79540 Loerrach (DE); KUHNY, Kurt, CH-4206 Seewen (CH); LEUENBERGER, Bruno, CH-4123 Allschwil (CH)
(74) Representative: Teipel, Stephan
(86) International application number: PCT/EP2002/008819
(87) International publication number: WO 2003/015537

(56) References cited:
- WO-A-00/22939
- WO-A-00/70967
- US-A- 5 008 254
- US-B1- 6 248 375
- CARLE, R: "Trends in fruit processing" OBST-, GEMÜSE- UND KARTOFFELVERARBEITUNG, vol. 85, no. 3, 2000, pages 125-131, XP009002259

## Description

The present invention relates to novel compositions containing finely dispersed carotenoids and to a process for the preparation of such compositions. The novel compositions of this invention can be used as colorants or additives for food, beverages, animal feeds, cosmetics or drugs.

More particularly, the present invention relates to novel compositions comprising pectin obtainable from sugar beet, a triglyceride, and a carotenoid, to a process for preparing these compositions, their use as a colorant for food, beverages, animal feeds, cosmetics or drugs; and to food, beverages, animal feeds, cosmetics or drugs containing such compsitions.

WO 00/70967 A1 discloses a composition comprising colouring substance bodies, such as a carotenoid, that are coated with beet pectin, such as pectin derived from sugar beet.

Pectins are basically α, 1→4 linked polygalacturonic acids which are partially esterified by methyl groups and which can by obtained from plants such as citrus fruit, apples and sugar beet. The term "sugar beet pectin" as used herein denotes pectins obtainable from sugar beet which are characterized and distinguished from citrus and apple pectins in that secondary hydroxy groups are partially acetylated, and by a lack of gelling properties. While such pectins might also be produced from pears and potatoes the commercially readily available pectins of this type are made from sugar beet, e.g. as GENU Beta Pectin Type BETA from CP Kelco (Copenhagen Pectin A/S, DK-4623 Lille Skensved, Denmark). Thus, the term "sugar beet pectin" is intended to denote all pectins having substantially the properties of pectin obtained from sugar beet and comprises pectins obtained from other sources, e.g., pears and potatoes inasmuch as they have substantially the properties of pectins obtained from sugar beet. For the the purpose of the present invention, the sugar beet pectins preferably are those of which a 10 wt.-% aqueous solution has a viscosity of 20 to 10000 mPa·s at 50 °C. The average molecular weight of such pectins is assumed to be in the range of 5 to 150 kDalton although this figure is not to be regarded as crucial in view of the well-known problematics of methodology in determinations of molecular weight.

The term "carotenoid" as used herein comprises a carotene or structurally related polyene compound which can be used as a colorant for food, beverages, animal feeds, cosmetics or drugs. Examples of such carotenoids are α- or β-carotene, 8'-apo-β-carotenal, 8'-apo-β-carotenoic acid esters such as the ethyl ester, canthaxanthin, astaxanthin, lycopene, lutein, zeaxanthin or crocetin, or mixtures thereof. The preferred carotenoid is β-carotene.

In the compositions of the present invention, the amount of sugar beet pectin is from about 0.5 to about 60.0 wt.-% and the amount of carotenoid is from about 0.1 to about 20.0 wt.-%.

Suitably, the novel compositions of this invention further contain adjuvants and/or excipients such as one or more of a mono- di-, oligo- or polysaccharide, a water-soluble antioxidant, a fat-soluble antioxidant, silicic acid and water.

Examples of mono- and disaccharides which may be present in the compositions of the present invention are saccharose, invert sugar, glucose, fructose, lactose and maltose. Examples of oligo- or polysaccharides which may be present in the compositions of the present invention are starch and starch hydrolysates, such as dextrins and maltodextrins, especially such in the range of 5-65 dextrose equivalents (hereinafter: DE) and glucose syrup, especially such in the range of 20-95 DE. The term "dextrose equivalent" (DE) denotes the degree of hydrolysation and is measure for the amount of reducing sugar calculated as D-glucose based on dry weight. Native starch has DE close to 0 while glucose has a DE = 100.

The triglyceride is suitably a vegetable oil or fat, such as corn oil, sunflower oil, soybean oil, safflower oil, rape seed oil, arachis oil, palm oil, palm kernel oil, cotton seed oil or cocos oil.

The water-soluble antioxidant may be ascorbic acid and salts thereof, e.g., sodium ascorbate, and the like. The fat-soluble antioxidant may be a tocopherol, e.g., dl-α-tocopherol (i.e., synthetic tocopherol), d-α-tocopherol (i.e., natural tocopherol), β- and γ-tocopherol and mixtures thereof; ascorbic acid esters of fatty acids such as ascorbyl palmitate or stearate; butyl hydroxy toluene; butyl hydroxy anisol; propyl gallate; or t-butyl hydroxy quinoline.

The compositions of the present invention may be an aqueous emulsion (i.e. an oil-in-water dispersion) or a powder.

In one aspect the present invention relates to solid compositions, i.e. stable, water-soluble or dispersible powders. In another aspect, the present inventions relates to liquid compositions, i.e., aqueous colloidal solutions or oil-in-water dispersions of such powders.

Typically, a powder composition according to the present invention comprises
about 1 to about 60 wt.-% , preferably about 5 to about 30 wt.-% of pectin;
about 0.2 to about 20 wt.-% preferably about 0.5 to about 10 wt.-% of a carotenoid;
0 to about 70 wt.-% preferably about 0 to about 40 wt.-% of a mono- or disaccharide;
0 to about 50 wt.-% preferably about 0 to about 35 wt.-% of starch;
0 to about 70 wt.-% preferably about 0 to about 40 wt.-% of a starch hydrolysate;
about 0.5 to about 50 wt.-% preferably about 1.5 to about 30 wt.-% of a triglyceride;
0 to about 5 % preferably about 0.5 to about 2 wt.-% of a water-soluble anti-oxidant;
0 to about 5 % preferably about 0.01 to about 2 wt.-% of a fat-soluble anti-oxidant;
0 to about 2 wt.-% preferably about 1 wt.-% of silicic acid; and
0 to about 10 wt.-% preferably about 1 to about 5 wt.-% of water;
the percentages of all ingredients totalling 100.

In accordance with the invention, the novel carotenoid compositions can be prepared by processing the ingredients in a manner known per se for the preparation of water-soluble or dispersible carotenoid compositions. Thus, the compositions can be prepared by a process which comprises homogenizing an aqueous solution or colloidal solution of the pectin and optional water-soluble excipients and adjuvants, a solution or dispersion of the carotenoid and optional fat-soluble adjuvants in a triglyeride and, if required, converting the dispersion obtained into a powder.

Typically, sugar beet pectin and optional water-soluble excipients and adjuvants are dissolved in water. The carotenoid and optional fat-soluble excipients and adjuvents are dissolved or suspended in triglyceride. The carotenoid solution (or dispersion) is then added to the aqueous pectin solution with stirring and the mixture is homogenized using conventional technology, e.g., by high-pressure homogenization, mixing devices as described in EP 1008380-A, high shear emulsification (rotor-stator systems), micronization or wet milling.

The so-obtained oil-in-water dispersion can be converted into a solid composition, e.g. a dry powder using conventional technology such spray-drying, spray drying in combination with fluidized-bed granulation (the latter technique commonly known as fluidized spray drying or FSD), or by a powder-catch technique where sprayed emulsion droplets are caught in a bed of an absorbant such as starch and subsequently dried.

The novel compositions of this invention can find use as colorants or vitamin A supplement for food, beverages, animal feeds, cosmetics or drugs. By the present invention there are preferably provided compositions comprising β-carotene as a colouring agent.

These compositions, when dissolved, dispersed or diluted in/with water to a final β-carotene concentration of 10 ppm are typically characterized by ultraviolet/visible-spectroscopy using deionized water as reference. At a sample thickness of 1 cm the dispersions show an extinction of at least 0.3 (preferably above 1.0) absorbance units at the wavelength of maximum optical density in the range of 400 to 600 nm. This is equivalent to a formal extinction coefficient of β-carotene in aqueous dispersion E(1%, 1cm) of 300 (preferably >1000).

The following Examples illustrate the invention further.

### Example 1

A dry premix of 80 g of beet pectin (GENU Pectin Type Beta of Copenhagen Pectin A/S; viscosity of a 10 % aqueous solution of the pectin at 50°C around 4000 mPa·s), 160 g of sucrose and 80 g of a maltodextrin (DE 20-23) was prepared. The dry premix was dissolved in 1200 ml of deionized water at 60°C and another 335 g of maltodextrin (DE 20-23) were added. After complete dissolution of the solids 8.0 g of Na-ascorbate was added to the mixture (= solution A).

136 g of a triglyceride (Durkex 500, partly hydrogenated soybean oil of Loders Croklaan B.V.; 1520 AA Wormerveer The Netherlands) and 0.9 g of dl-α-tocopherol were mixed and heated to 140°C. Subsequently, 11 g of β-carotene was suspended in the mixture of triglyceride and tocopherol. By stirring for about 10 minutes at 140°C a clear solution of β-carotene was obtained (= solution B).

Solution A was heated to 70°C and a crude emulsion was prepared by adding 135 g of solution B to solution A while gently stirring. A fine emulsion was obtained by a five passage high pressure homogenizing treatment of the preemulsion at a pressure of 50/300 bar (APV Lab Homogenizer Type Gaulin Lab 40-10 RBFI of APV Switzerland AG, CH-3076 Worb). The emulsion was diluted by adding an equal volume of deionized water at 60°C and then spray dried in a laboratory spray dryer (Mobile Minor of GEA Niro A/S, DK-2860 Söborg) at an inlet temperature of 200°C-210°C and an outlet temperature of 70-75°C. The spray-dried powder was dried in a vacuum oven at room temperature over night.

A fine powder was obtained with a water content of 2.2 %. The β-carotene content of the powder was 1.1 % as determined by spectrophotometry and HPLC-analysis. The powder was dispersed in deionized water and the extinction of the dispersion was measured in a 1 cm quartz precision cell against water. For a 10 ppm dispersion of β-carotene an extinction of 2.109 at a wavelength of 464 nm was calculated (E(1%,1cm) =2109)

### Example 2

A dry premix of 160 g of beet pectin (Copenhagen Pectin A/S; viscosity of a 10 % aqueous solution of the pectin at 50°C around 500 mPa·s), 160 g of sucrose and 335 g of a maltodextrin (DE 20-23) was prepared. The dry premix was dissolved in 1400 ml of deionized water at 60°C. After complete dissolution of the solids 8.0 g of Na-ascorbate was added to the mixture (= solution A).

136 g of a triglyceride (Durkex 500) and 0.9 g of dl-α-tocopherol were mixed and heated to 140°C. Subsequently, 11 g of β-carotene was suspended in the mixture of triglyceride and tocopherol. By stirring for about 10 minutes at 140°C a clear solution of β-carotene was obtained (= solution B).

Solution A was heated to 70°C and a crude emulsion was prepared by adding 135 g of solution B to solution A while gently stirring. A fine emulsion was obtained by a three passage high pressure homogenizing treatment of the preemulsion at a pressure of 50/300 bar (APV Lab Homogenizer Type Gaulin Lab 40-10 RBFI). The emulsion was diluted by adding an equal volume of deionized water at 60°C and then spray dried in a laboratory spray drier (Mobile Minor of GEA Niro A/S) at an inlet temperature of 200°C-210°C and an outlet temperature of 70-75°C. The spray dried powder was dried in a vacuum oven at room temperature over night.

A fine powder was obtained with a water content of 2.5 %. The β-carotene content of the powder was 1.2 % as determined by spectrophotometry and HPLC-analysis. The powder was dispersed in deionized water and the extinction was measured in a 1 cm quartz precision cell against water. For a 10 ppm dispersion of β-carotene an extinction of 2.051 at a wavelength of 463 nm was calculated (E(1%,1cm)=2051).

### Example 3

A dry premix of 28.6 g beet pectin (GENU Pectin Type Beta of Copenhagen Pectin A/S; viscosity of a 10 % aqueous solution of the pectin at 50°C around 4000 mPa·s) and 121.4 g sucrose was prepared. The dry premix was dissolved in 180 ml of deionized water at 50°C for 30 minutes under stirring (= solution A).

A 30 % suspension of β-carotene in corn oil stabilized by dl-α-tocopherol (β-Carotene 30 % FS of Roche Vitamins) was heated under stirring for about 30 minutes at a temperature of 160°C (= solution B).

An emulsion was prepared by adding solution B to solution A. By vigorously stirring for 30 minutes at 50°C a fine emulsion was obtained. The emulsion was diluted by adding 200 ml of deionized water.

300 g of the diluted emulsion were taken and, again, diluted with 50 ml of water. The final emulsion was sprayed into a cooled fluidized bed of corn starch. Excess corn starch was removed by sieving and a coarse powder was obtained. The powder was dried in an air stream at room temperature for about 2 hours.

A powder was obtained with a water content of 6.4 %. The β-carotene content of the powder was 2.5 % as determined by spectrophotometrical assay. The starch content of the powder was 54 %. The powder was dispersed in deionized water and the extinction was measured in a 1 cm quartz precision cell against water. For a 10 ppm dispersion of β-carotene an extinction of 0.401 at a wavelength of 530 nm was calculated (E(1%,1cm) =401).

### Example 4

Instant beverage powders were prepared according to the following compositions:

| Ingredients | # 1 [g] | # 2 [g] |
|---|---|---|
| Sucrose, fine crystalline | 920.0 | 920.0 |
| Ascorbic acid, fine powder | 2.0 | 2.0 |
| Citric acid anhydrous, powder | 55.0 | 55.0 |
| Orange flavor¹ | 7.0 | 7.0 |
| Tri-Na citrate anhydrous | 6.0 | 6.0 |
| Tri-Ca phosphate | 5.0 | 5.0 |
| powder according Ex. 1 | 5.0 | - |
| powder according Ex. 2 | - | 5.0 |

| | | |
|---|---|---|
| ¹ e.g. Orange Flavor 76905-71 from Givaudan Duebendorf Ltd | | |

### Procedure:

All ingredients were sieved through a 0.7 mm sieve.

The sieved ingredients were blended in a turbula mixer for 20 minutes

### Example 5

Instant pudding powders were prepared according to the following compositions:

| Ingredients | # 1 [g] | # 2 [g] |
|---|---|---|
| Sucrose, fine crystalline | 840.0 | 840.0 |
| Corn starch, cold swelling | 129.0 | 129.0 |
| Stabilizer¹ | 23.0 | 23.0 |
| Vanilla flavor² | 4.0 | 4.0 |
| powder according Example 1 | 4.0 | - |
| powder according Example 2 | - | 4.0 |

| | | |
|---|---|---|
| ¹ e.g. Flanogen ADG 56 from SKW Biosystems ² e.g. Vanilla flavor 75016-32 from Givaudan Dubendorf Ltd | | |

### Procedure:

All ingredients were sieved through a 0.7 mm sieve.

The sieved ingredients were blended in a turbula mixer for 20 min.

## Claims

1. A composition comprising pectin obtainable from sugar beet, a triglyceride, a carotenoid and, optionally, adjuvants and/or excipients, wherein the amount of pectin is from about 0.5 to about 60.0 wt.-% and the amount of carotenoid is from about 0.1 to about 20.0 wt.-%.

2. A composition as in claim 1 wherein the carotenoid is α- or β-carotene, 8'-apo-β-carotenal, 8'-apo-β-carotenoic acid ethyl ester, canthaxanthin, astaxanthin, lycopene, lutein, zeaxanthin or crocetin or mixtures thereof.

3. A composition as in claim 2 wherein the carotenoid is β-carotene.

4. A composition as in any one of claims 1-3 wherein the pectin is one of which a 10 wt.-% aqueous solution has a viscosity of 20 to 10000 mPa·s at 50 °C.

5. A composition as in any one of claims 1-4 wherein at least one of a mono- di-, oligo- or polysaccharide, a water-soluble anti-oxidant, a fat-soluble anti-oxidant, silicic acid and water are additionally present.

6. A composition as in claim 5 wherein the mono- or disaccharide is saccharose, invert sugar, glucose, fructose, lactose or maltose.

7. A composition as in claim 5 wherein the polysaccharide is a starch or a starch hydrolysate.

8. A composition as in claim 7 wherein the starch hydrolysate is a dextrin or a maltodextrin (in the range of 5-65 dextrose equivalents) or a glucose syrup (in the range of 20-95 dextrose equivalents).

9. A composition as in claim 1 wherein the triglyceride is a vegetable oil or fat.

10. A composition as in any one of claims 1-9 which is a powder.

11. A composition as in claim 10 which comprises
about 1 to about 60 wt.-% of pectin obtainable from sugar beet;
about 0.2 to about 20 wt.-% of a carotenoids;
0 to about 70 wt.-% of a mono- or disaccharide;
0 to about 50 wt.-% of starch;
0 to about 70 wt.-% of a starch or a starch hydrolysate;
about 0.5 to about 50 wt.-% of a triglyceride;
0 to about 5 % of a water-soluble anti-oxidant;
0 to about 5 % of a fat-soluble anti-oxidant;
0 to about 2 wt.-% of silicic acid; and
0 to about 10 wt.-% of water.

12. A composition as in any one of claims 1-9 which is an oil-in-water dispersion.

13. A composition as in claim 12 which comprises
about 0.5 to about 30 wt.-% of pectin obtainable from sugar beet,
about 0.1 to about 10 wt.-% of a carotenoid,
0 to about 35 wt.-% of a mono- or disaccharide,
0 to about 35 wt.-% of a starch or a starch hydrolysate,
about 0.25 to about 25 wt.-% of a triglyceride,
0 to about 2.5 % of a water-soluble anti-oxidant,
0 to about 2.5 % of a fat-soluble anti-oxidant, and
5 to about 95 wt.-% of water.

14. A composition as in any one of claims 11-13 which when dissolved, dispersed or diluted with/in water to a final β-carotene concentration of 10 ppm has an extinction coefficient E(1%, 1cm) of ≥ 300 at the extinction maximum.

15. A process for the preparation of a composition as claimed in any one of claims 1-14 which comprises homogenizing, in an aqueous solution or colloidal solution of the pectin and optional water-soluble excipients and adjuvants, a solution or dispersion of the carotenoid and optional fat-soluble adjuvants in a triglyeride and, if required, converting the dispersion obtained into a powder.

16. The use of a composition as claimed in any one of claims 1-14 as a colorant for food, beverages, animal feeds, cosmetics or drugs.

17. Food, beverages, animal feeds, cosmetics or drugs containing a composition as claimed in any one of claims 1-14.

## Patentansprüche

1. Zusammensetzung, umfassend Pectin, erhältlich aus Zuckerrüben, ein Triglycerid, ein Carotinoid und gegebenenfalls Adjuvanzien und/oder Hilfsstoffe, wobei die Menge an Pectin etwa 0,5 bis etwa 60,0 Gew.-% beträgt und die Menge an Carotinoid etwa 0,1 bis etwa 20,0 Gew.-% beträgt.

2. Zusammensetzung nach Anspruch 1, wobei das Carotinoid α- oder β-Carotin, 8'-apo-β-Carotinal, 8'-apo-β-Carotinsäureethylester, Canthaxanthin, Astaxanthin, Lycopin, Lutein, Zeaxanthin oder Crocetin oder Gemische davon ist.

3. Zusammensetzung nach Anspruch 2, wobei das Carotinoid β-Carotin ist.

4. Zusammensetzung nach einem der Ansprüche 1 bis 3, wobei das Pectin eines ist, dessen 10gew.-%ige wässerige Lösung bei 50 °C eine Viskosität von 20 bis 10.000 mPa · s hat.

5. Zusammensetzung nach einem der Ansprüche 1 bis 4, wobei außerdem mindestens eines von einem Mono-, Di-, Oligo- oder Polysaccharid, einem wasserlöslichen Antioxidationsmittel, einem fettlöslichen Antioxidationsmittel, Kieselsäure und Wasser vorliegt.

6. Zusammensetzung nach Anspruch 5, wobei das Mono- oder Disaccharid Saccharose, Invertzucker, Glucose, Fructose, Lactose oder Maltose ist.

7. Zusammensetzung nach Anspruch 5, wobei das Polysaccharid Stärke oder ein Stärkehydrolysat ist.

8. Zusammensetzung nach Anspruch 7, wobei das Stärkehydrolysat ein Dextrin oder ein Maltodextrin (im Bereich von 5 - 65 Dextroseäquivalenten) oder ein Glucosesirup (im Bereich von 20 - 95 Dextroseäquivalenten) ist.

9. Zusammensetzung nach Anspruch 1, wobei das Triglycerid ein pflanzliches Öl oder Fett ist.

10. Zusammensetzung nach einem der Ansprüche 1 bis 9, welche ein Pulver ist.

11. Zusammensetzung nach Anspruch 10, umfassend
etwa 1 bis etwa 60 Gew.-% Pectin, erhältlich aus Zuckerrüben;
etwa 0,2 bis etwa 20 Gew.-% eines Carotinoids;
0 bis etwa 70 Gew.-% eines Mono- oder Disaccharids;
0 bis etwa 50 Gew.-% Stärke;
0 bis etwa 70 Gew.-% einer Stärke oder eines Stärkehydrolysats;
etwa 0,5 bis etwa 50 Gew.-% eines Triglycerids;
0 bis etwa 5 Gew.-% eines wasserlöslichen Antioxidationsmittels;
0 bis etwa 5 Gew.-% eines fettlöslichen Antioxidationsmittels;
0 bis etwa 2 Gew.-% Kieselsäure und
0 bis etwa 10 Gew.-% Wasser.

12. Zusammensetzung nach einem der Ansprüche 1 bis 9, welche eine Öl-in-Wasser-Dispersion ist.

13. Zusammensetzung nach Anspruch 12, umfassend
etwa 0,5 bis etwa 30 Gew.-% Pectin, erhältlich aus Zuckerrüben;
etwa 0,1 bis etwa 10 Gew.-% eines Carotinoids;
0 bis etwa 35 Gew.-% eines Mono- oder Disaccharids;
0 bis etwa 35 Gew.-% einer Stärke oder eines Stärkehydrolysats;
etwa 0,25 bis etwa 25 Gew.-% eines Triglycerids;
0 bis etwa 2,5 Gew.-% eines wasserlöslichen Antioxidationsmittels;
0 bis etwa 2,5 Gew.-% eines fettlöslichen Antioxidationsmittels und
5 bis etwa 95 Gew.-% Wasser.

14. Zusammensetzung nach einem der Ansprüche 11 - 13, die, wenn sie mit/in Wasser auf eine β-Carotinendkonzentration von 10 ppm gelöst, dispergiert oder verdünnt ist, einen Extinktionskoeffizienten E(1 %, 1 cm) von ≥ 300 am Extinktionsmaximum hat.

15. Verfahren zur Herstellung einer Zusammensetzung nach einem der Ansprüche 1 - 14, welches das Homogenisieren einer Lösung oder Dispersion des Carotinoids und gegebenenfalls von fettlöslichen Adjuvanzien in einem Triglycerid in einer wässerigen Lösung oder kolloiden Lösung des Pectins und gegebenenfalls wasserlöslichen Hilfsstoffen und Adjuvanzien und, wenn gewünscht, das Umwandeln der erhaltenen Dispersion in ein Pulver umfasst.

16. Verwendung einer Zusammensetzung nach einem der Ansprüche 1 - 14 als Färbemittel für Lebensmittel, Getränke, Tierfutter, Kosmetika oder Arzneimittel.

17. Lebensmittel, Getränke, Tierfutter, Kosmetika oder Arzneimittel, enthaltend eine Zusammensetzung nach einem der Ansprüche 1 - 14.

## Revendications

1. Composition comprenant une pectine pouvant être obtenue à partir de betterave à sucre, un triglycéride, un caroténoïde et, de manière facultative, des adjuvants et/ou des excipients, dans laquelle la quantité de pectine est comprise entre environ 0,5 et environ 60,0 % en poids, et la quantité de caroténoïde est comprise entre environ 0,1 et environ 20,0 % en poids.

2. Composition suivant la revendication 1, dans laquelle le caroténoïde est le α-carotène ou le β-carotène, le β-apo-8'-caroténal, l'ester éthylique de l'acide caroténoïque, la canthaxanthine, l'astaxanthine, le lycopène, la lutéine, la zéaxanthine ou la crocétine ou des mélanges de ces corps.

3. Composition suivant la revendication 2, dans laquelle le caroténoïde est le β-carotène.

4. Composition suivant l'une des revendications 1 à 3, dans laquelle la pectine est une des pectines dont une solution aqueuse à 10 % en poids présente une viscosité de 20 à 10.000 mPas à 50°C.

5. Composition suivant l'une des revendications 1 à 4, dans laquelle au moins un monosaccharide, disaccharide, oligosaccharide ou polysaccharide, un antioxydant soluble dans l'eau, un antioxydant soluble dans les graisses, de l'acide silicique et de l'eau sont en outre présents.

6. Composition suivant la revendication 5, dans laquelle le monosaccharide ou le disaccharide est le saccharose, le sucre inverti, le glucose, le fructose, le lactose ou le maltose.

7. Composition suivant la revendication 5, dans laquelle le polysaccharide est un amidon ou un hydrolysate d'amidon.

8. Composition suivant la revendication 7, dans laquelle l'hydrolysate d'amidon est une dextrine ou une maltodextrine (dans le domaine allant de 5 à 65 équivalents dextrose) ou un sirop de glucose (dans le domaine allant de 20 à 95 équivalents dextrose).

9. Composition suivant la revendication 1, dans laquelle le triglycéride est une huile ou une graisse végétale.

10. Composition suivant l'une quelconque des revendications 1 à 9, **caractérisée en ce qu'**elle est une poudre.

11. Composition suivant la revendication 10, comprenant
entre environ 1 et environ 60 % en poids de pectine pouvant être obtenue à partir de betterave à sucre ;
entre environ 0,2 et environ 20 % en poids d'un caroténoïde ;
entre 0 et environ 70 % en poids d'un monosaccharide ou d'un disaccharide ;
entre 0 et environ 50 % en poids d'amidon ;
entre 0 et environ 70 % en poids d'un amidon ou d'un hydrolysate d'amidon ;
entre environ 0,5 et environ 50 % en poids d'un triglycéride ;
entre 0 et environ 5 % d'un antioxydant soluble dans l'eau ;
entre 0 et environ 5 % d'un antioxydant soluble dans les graisses ;
entre 0 et environ 2 % en poids d'acide silicique ; et
entre 0 et environ 10 % en poids d'eau.

12. Composition suivant l'une quelconque des revendications 1 à 9, **caractérisée en ce qu'**elle est une dispersion huile-dans-eau.

13. Composition suivant la revendication 12, comprenant
entre environ 0,5 et environ 30 % en poids de pectine pouvant être obtenue à partir de betterave à sucre ;
entre environ 0,1 et environ 10 % en poids d'un caroténoïde ;
entre 0 et environ 35 % en poids d'un monosaccharide ou d'un disaccharide ;
entre 0 et environ 35 % en poids d'un amidon ou d'un hydrolysate d'amidon ;
entre environ 0,25 et environ 25 % en poids d'un triglycéride ;
entre 0 et environ 2,5 % d'un antioxydant soluble dans l'eau ;
entre 0 et environ 2,5 % d'un antioxydant soluble dans les graisses ; et
entre 5 et environ 95 % en poids d'eau.

14. Composition suivant l'une quelconque des revendications 11 à 13, qui, lorsqu'elle est dissoute, dispersée ou diluée avec/dans l'eau jusqu'à obtention d'une concentration finale en β-carotène de 10 ppm, présente un coefficient d'extinction E(1%, 1cm) supérieur ou égal à 300 à son niveau maximum d'extinction.

15. Procédé pour la préparation d'une composition suivant l'une quelconque des revendications 1 à 14, comprenant l'homogénéisation, dans une solution aqueuse ou une solution colloïdale, de la pectine, et de manière facultative, des excipients et des adjuvants solubles dans l'eau, d'une solution ou d'une dispersion du caroténoïde et, de manière facultative, des adjuvants solubles dans les graisses dans un triglycéride et, si nécessaire, la transformation de la dispersion obtenue en poudre.

16. Utilisation d'une composition suivant l'une quelconque des revendications 1 à 14 comme colorant pour aliments, boissons, aliments pour animaux, cosmétiques ou médicaments.

17. Aliments, boissons, aliments pour animaux, cosmétiques ou médicaments contenant une composition suivant l'une quelconque des revendications 1 à 14.
